# EUROPEAN PATENT SPECIFICATION

(11) **EP 2 814 437 B1**
(45) Date of publication and mention of the grant of the patent: **25.05.2016**
(21) Application number: 13713240.3
(22) Date of filing: 11.02.2013
(51) Int. Cl.: A61F 13/15

(54) **MACHINE FOR MAKING ABSORBENT SANITARY ARTICLES**
MASCHINE ZUR HERSTELLUNG SAUGFÄHIGER HYGIENEARTIKEL
MACHINE POUR FABRIQUER DES ARTICLES SANITAIRES ABSORBANTS

(30) Priority: 16.02.2012 IT BO20120071
(43) Date of publication of application: 24.12.2014
(73) Proprietor: GDM S.p.A., 40133 Bologna (IT)
(72) Inventor: PIANTONI, Matteo, I-24021 Albino (Bergamo) (IT); PEREGO, Alberto, I-20154 Milano (IT)
(74) Representative: Bianciardi, Ezio
(86) International application number: PCT/IB2013/051111
(87) International publication number: WO 2013/121338

(56) References cited:
- WO-A1-01/56525
- WO-A1-2007/105938
- US-A1- 2003 169 433
- US-A1- 2004 030 432
- US-A1- 2010 305 740

## Description

### Technical field

This invention relates to a machine for making absorbent sanitary articles.

More specifically, the invention relates to a machine for making absorbent sanitary articles such as nappies for babies and incontinence pads for adults, sanitary towels or the like.

### Background art

US 2010/305740 discloses a system and a method for detecting and rejecting defective absorbent articles from a converting line along a machine direction of the system.

As is known, a machine for making absorbent sanitary articles has a production line along which there advances a continuous web of absorbent material consisting of a sheet of permeable material (nonwoven fabric) superposed on a sheet of impermeable material, with absorbent padding interposed between the two sheets. As the continuous web advances along the production line, additional components are applied to the continuous web, such as, for example, lateral stretch bands, lateral sealing flaps, a rear stretch tape and a front band designed to engage the lateral sealing flaps.

Once these additional components have been applied to the continuous web, a continuous sequence of absorbent sanitary articles is formed and a cutting device located downstream of the production line divides the continuous sequence into individual absorbent articles which are then folded and packaged.

Every line for the production of absorbent sanitary articles also comprises a rejection station, located downstream of the cutting device, which rejects defective articles, that is to say, absorbent sanitary articles which do not meet specified quality parameters.

Checking for defective absorbent sanitary articles is carried out by a logic built into a programmable electronic controller of the machine for making the articles.

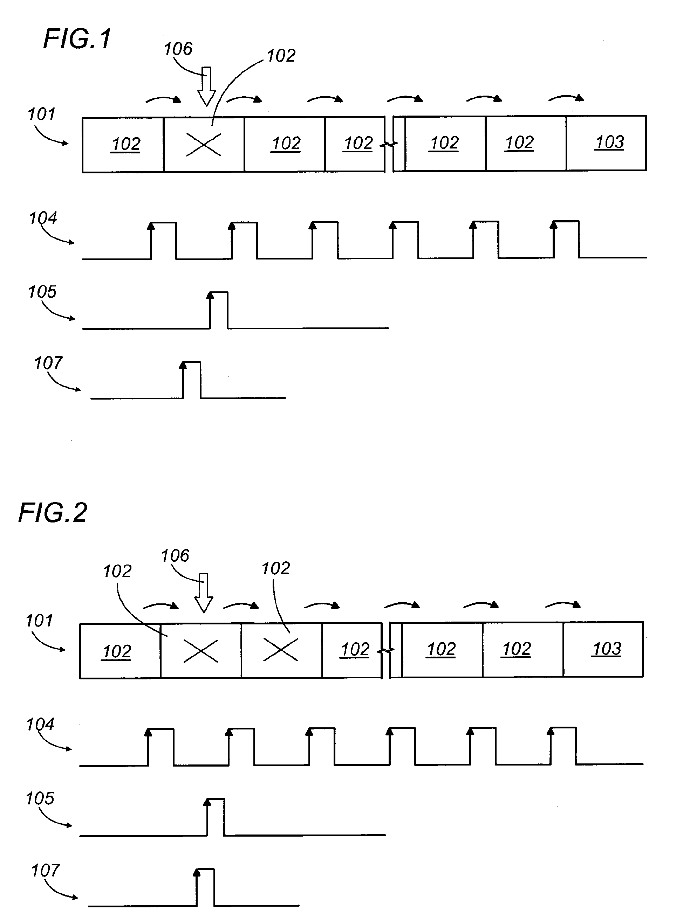

The electronic controller (as illustrated in Figure 1) assigns information about the article being processed to a shift register 101 (in the electronic controller memory) on which the electronic controller operates by means of a shift command 107. The shift register 101 is defined by a predetermined number of steps 102, each associated with a time position of an absorbent article being processed along the production line. In other words, each step 102 corresponds to a block (memory location) of the electronic controller shift register 101. Associated with each block there is a sector (a segment with substantially constant length) of the production line, and the data content of a block relates to the absorbent sanitary article present in that sector of the production line in the time interval of the respective step 102.

A prior art system for identifying defective absorbent articles along the production line is to place an optical inspection system upstream of a final device for cutting the continuous sequence of absorbent sanitary articles. The optical inspection system is connected to the above-mentioned electronic controller and is designed to detect and flag any production defects in each absorbent article being made. In particular, at the moment when the optical inspection system detects a production defect, it signals it to the electronic controller which assigns reject information (indicated with an "X" in the accompanying drawings) to a predetermined step 102 of the shift register 101, that is to say, the electronic controller uses a write operation 106 to "mark" the corresponding block of the shift register 101.

The information contained in that block will shift in the shift register until reaching a final position 103 (the last block in the shift register) corresponding to the rejection station of the production line where the defective absorbent article will be rejected.

The optical inspection systems are controlled by a trigger pulse 104 generated by the machine electronic controller.

Once the trigger pulse 104 has been received, the optical inspection system captures an image of the absorbent article, analyses it using a programmed procedure and, when a response time which is less than a predetermined maximum has elapsed, indicates any defect by means of a response signal 105 generated by the optical inspection system itself.

The optical inspection system maximum response time is known and is closely linked to the technology implemented in the optical inspection system: that allows the programmer to set the moment for activation of the shift command 107 after the optical inspection system maximum response time. In this way, the steps 102 of the shift register 101 always shift after the response signal 105 and the electronic controller can always correctly assign the reject information "X" to the block which is in a predetermined position of the register 101, that is to say, to the block which is in the position corresponding to the sector of the production line in which the optical inspection system is located and in which the defective article is present at the step 102.

The optical inspection system is a commercial device for which the maker indicates a maximum response time in the technical specifications.

However, during machine operation, the optical inspection system response signal may be subject to unforeseen delays caused by an increase in the response time basically due to additional operations for analysing images which a machine operator may request of the optical inspection system by means of an operator interface connected to the electronic controller and which overload the optical inspection system.

The increase in the response time may result in a delay between the optical inspection system response signal 105 and the shift register 101 shift command 107, or a simultaneous response signal 105 and shift register 101 shift command 107, as illustrated respectively in Figures 1 and 2.

If the optical inspection system response signal 105 comes after the shift register 101 shift command 107, the electronic controller assigns the reject information "X" after the shift register 101 has shifted, that it so say, it assigns the "X" to the absorbent article after the article inspected, as illustrated in Figure 1. In this way, at the rejection station 103, the defective absorbent article will not be rejected, whilst the absorbent article after the defective one will be rejected even if it is free of defects.

If the optical inspection system response signal is simultaneous with the shift register 101 shift command 107, the electronic controller assigns the reject information "X" to more than one step 102 of the shift register 101 since the electronic controller is uncertain in its identification of the step 102 of the absorbent article inspected, as shown in Figure 2. In this way, at the rejection station, both the defective absorbent article and the absorbent article after the defective one will be rejected, even if the latter is free of defects.

From the above it may be inferred that, if the optical inspection system does not comply with the maximum response time limit due to any unforeseen delays, the electronic controller does not guarantee assignment of the reject information to the correct step 102 of the shift register 101, corresponding to the absorbent article to be rejected.

### Disclosure of the invention

The aim of this invention is to overcome the above-mentioned disadvantages by providing a machine for making absorbent sanitary articles which can correctly assign the reject information to the step of the shift register corresponding to the time position of the defective absorbent article.

The above-mentioned technical purpose and the aims are achieved by a machine and a method having the technical features described in the respective independent claim 1 and 8.

### Brief description of the drawings

Further features and advantages of this invention are more apparent in the description below, with reference to a preferred, non-limiting embodiment of a machine as illustrated in the accompanying drawings, in which:
Figure 1 illustrates check signals and information in a prior art machine for making articles, in a first configuration;
Figure 2 illustrates check signals and information in a prior art machine for making articles, in a second configuration;
Figure 3 is a schematic front view of a machine for making articles according to this invention;
Figure 4 is a block diagram of a first embodiment of a check section of the machine of Figure 3;
Figure 5 is a block diagram of a second embodiment of a check section of the machine of Figure 3;
Figure 6 shows a series of check and control signals generated by the check section of Figure 5.

### Detailed description of the preferred embodiments of the invention

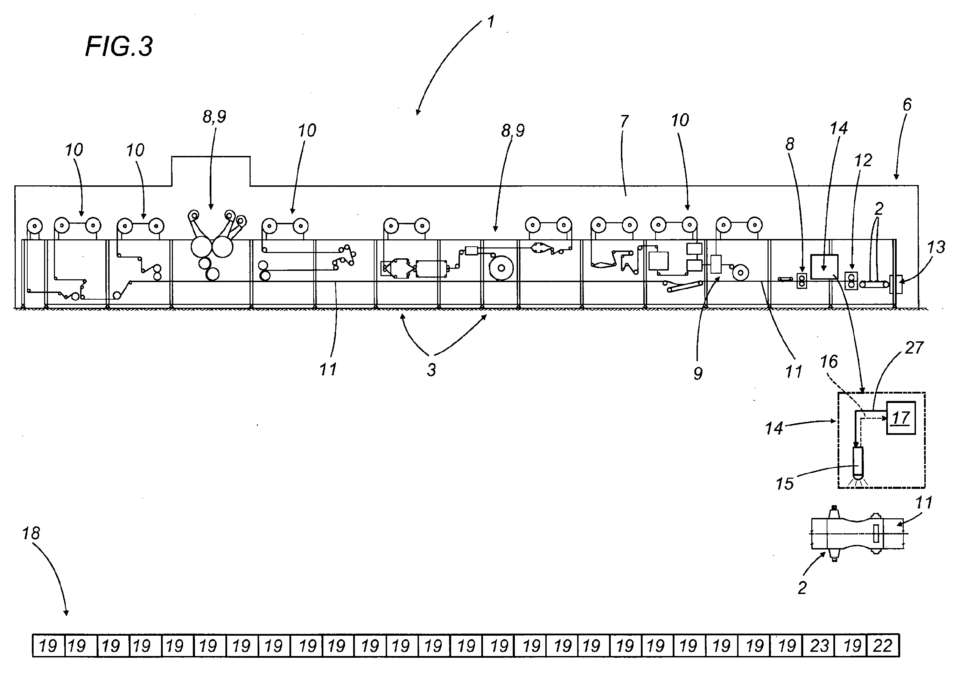

With reference to Figure 3, the numeral 1 denotes a machine for making absorbent sanitary articles 2 comprising a substantially horizontal production line 3 along which the absorbent sanitary article 2 is made.

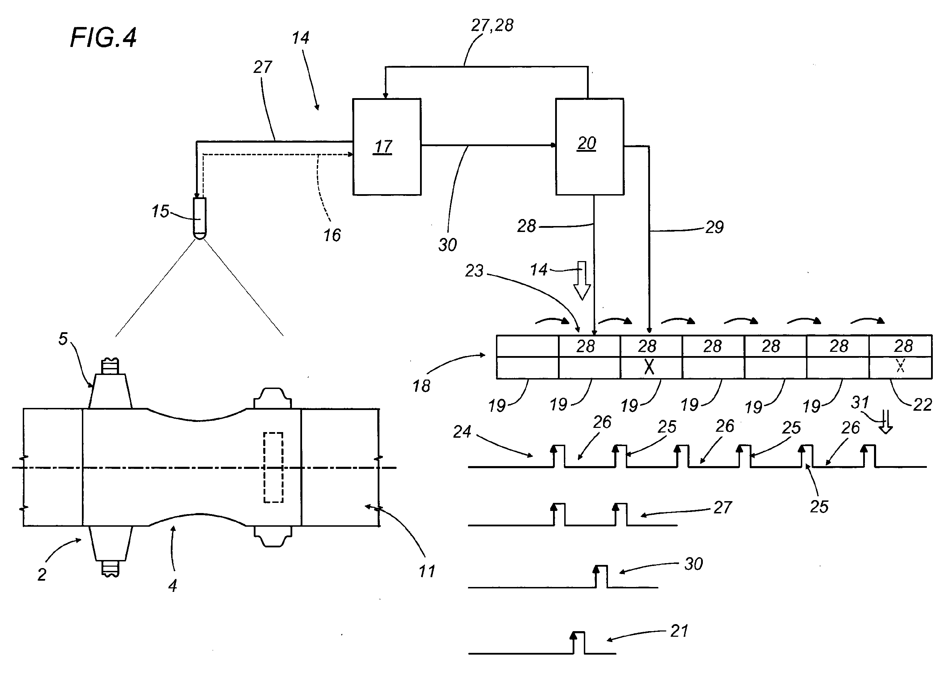

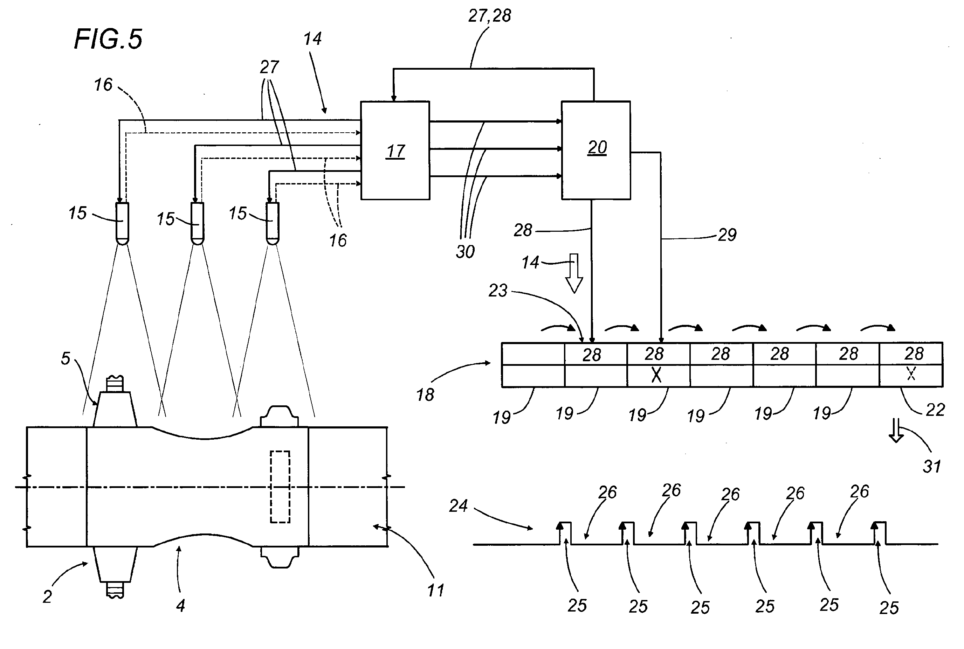

In particular, the absorbent sanitary article 2 comprises a plurality of components, which can be divided into basic components 4 and additional components 5 (Figures 4 and 5).

The machine 1 for making articles is a cyclic machine of the continuous type, that is to say, with each machine cycle, a finished absorbent sanitary article 2 is made.

The machine 1 comprises a base 6 substantially in the shape of a parallelepiped and delimited at the front by a vertical wall 7.

The vertical wall 7 is the support for a plurality of operating units comprising cutting devices and sealing devices 8 and 9, as well as units 10 for unwinding reels of the various materials used to make the article 2, the reels being mounted on axes which are transversal to the wall 7 and designed to produce a continuous sequence 11 of articles 2 using the basic and additional components 4 and 5.

The cutting devices and sealing devices 8 and 9 and the unwinding units 10 forming the machine 1 operating units are of the known type.

Downstream of the operating units 8, 9 and 10, a cutting unit 12 transversally separates the continuous sequence 11 into individual articles 2.

The individual articles 2, separated from the continuous sequence 11, are conveyed towards a rejection station 13, located at the end of the production line 3, downstream of the cutting unit 12, which rejects defective articles 2 using known ejection methods. Articles 2 which are free of defects are, in contrast, conveyed towards subsequent packaging steps (not illustrated).

The machine 1 for making articles comprises at least one vision system 14 for checking the quality of the articles 2.

The vision system 14 is designed to detect and signal any production defects in the article 2, forming a machine 1 optical inspection system.

The vision system 14 is located at the end of the production line 3, so that it analyses the absorbent article 2 complete with the basic components 4 and the additional components 5. In particular, the vision system 14 is located facing the continuous sequence 11 of finished articles 2 immediately upstream of the cutting unit 12. Alternatively, the vision system 14 is located downstream of the cutting unit 12, facing each individual finished article 2 separated from the continuous sequence 11.

The vision system 14 comprises at least a video camera 15 for capturing at least one image 16 of an article 2 and a unit 17 for acquiring and processing images 16 of the article 2 and connected to the video camera 15.

In the preferred embodiment, the image capturing video camera 15 is a linear video camera.

As the continuous sequence 11 advances along the production line 3, the video camera 15 inspects each article 2 with a field of vision which spans the entire cross-section of the article 2.

The image acquisition and processing unit 17 analyses the image 16 and thereby assesses whether or not the preset quality parameters of the absorbent article 2 fall within respective tolerance ranges. If one or more of the quality parameters are outside the respective tolerance range, the acquisition and processing unit 17 signals the presence of a production defect in the absorbent article 2.

These production defects, once detected and signalled by the acquisition and processing unit 17, cause the absorbent article 2 inspected to be rejected when it reaches the rejection station 13 of the production line 3.

In terms of logical representation, the machine 1 for making articles can be divided into a sequence of adjacent sectors which are mapped by at least one shift register 18, as illustrated in Figure 3.

The shift register 18 (also called a "chain") is graphically represented as a sequence of consecutive rectangular blocks 19. Each block 19 of the register 18 is defined as a step of the "chain" and represents a segment of the production line 3.

Each block 19 of the shift register 18 corresponds to a time position of the absorbent article 2 as it passes through the above-mentioned operating stations 8, 9 and 10 of the machine 1 while it is being made up. In particular, the information relating to the state of the article 2 (or the component materials) being processed along the production line 3 is saved in the shift register 18.

The machine 1 for making articles is equipped with a programmable electronic machine controller device 20.

The electronic controller device 20 generates a machine synchronism signal 24. The synchronism signal 24 is made up of a train of control pulses 25 with a phase, a duration and a frequency which depend on the machine 1 processing cycle. In particular, two consecutive pulses 25 of the synchronism signal 24 define a machine step 26.

The electronic controller device 20 applies to the shift register 18 a shift command 21 correlated and synchronised with the synchronism signal 24.

Corresponding to and synchronised with each pulse 25 of the synchronism signal 24, the shift command 21 shifts forward one step 26 the information contained in each block 19, until it reaches the final block 22 of the "chain" when the shift corresponds to the moment when the finished absorbent article 2 enters the rejection station 13.

The position of the vision system 14 along the production line 3 has a predetermined correspondence in the shift register 18, in particular the vision system 14 corresponds to a position 23 of the register 18.

Therefore, in particular, between the block 19 at the position 23 and the final block 22 there is a predetermined number of blocks 19.

The vision system 14 is connected to the electronic controller device 20 and is activated by the electronic controller 20 by means of a trigger signal 27 generated by the electronic controller 20 synchronised with the machine synchronism signal 24.

The electronic controller device 20 also generates an identifier 28 which is uniquely associated with the article 2 being inspected by the vision system 14 within the same machine step 26.

The electronic controller device 20 assigns the identifier 28 to the block 19 of the shift register 18 which is located in the position 23 corresponding to the moment when the article 2 is inspected.

The identifier 28 is preferably a sequential number generated by an incremental counter, of the known type and not illustrated. Alternatively, the identifier 28 may be a string of alphanumeric characters generated by a calculation algorithm, of the known type and not illustrated.

Once the identifier 28 has been generated, the electronic controller device 20 sends the vision system 14 that identifier 28 with the respective trigger signal 27. In this way, the vision system 14 acquisition and processing unit 17 associates the identifier 28, generated by the electronic controller device 20, with the image 16 of the article 2 to be inspected obtained by the video camera 15 activated by the trigger signal 27.

When a response time T has elapsed, the vision system 14 acquisition and processing unit 17 generates a response signal 30. The response signal 30 is a message which if necessary comprises reject information 29 associated with the identifier 28.

The vision system 14 acquisition and processing unit 17 sends the response signal 30 to the electronic controller device 20.

At this point, the electronic controller device 20 associates the reject information 29 linked to the identifier 28 (present in the response signal 30) with the block 19 of the shift register 18 having the identifier 28, that is to say, the electronic controller device 20 "marks" the single block 19 in which the identifier 28 is saved.

When the content of the block 19 having the identifier 28 with which the reject information 29 is associated, shifting one step after another, reaches the final block 22 of the register 18, the electronic controller device 20 will generate a rejection command 31 for the final article 2 at the rejection station 13.

Therefore, in contrast to the prior art, the electronic controller device 20 no longer associates the reject information 29 in a predetermined position 23 of the register 18, but instead associates the reject information 29 with the corresponding identifier 28 assigned to a predetermined block 19 which advances in the register 18.

That logic allows the shift register 18 shift command 21 to be made independent of the vision system 14 response time T. In fact, the shift register 18 shift command 21 may come before, after or simultaneously with the vision system 14 response signal 30, since the reject information 29 present in the response signal 30 is not associated with a shift register 18 block 19 having a predetermined position 23, but is instead associated with the shift register 18 block 19 which has the identifier 28 present in the response signal 30, whatever the position of the identifier 28 in the shift register.

In order to inspect the article 2, the vision system 14 may use a single video camera 15 or a plurality of video cameras 15, each dedicated to a portion of the article 2.

With reference to Figure 5, three separate video cameras 15 are used, each dedicated to inspecting a respective portion of the article 2. The three video cameras 15 are activated within the same machine step 26 and the acquisition and processing unit 17 supplies the response signal 30 associated with acquisition and processing of the images 16 captured by all three of the video cameras 15 within the same machine step 26.

In this embodiment, when the image 16 processing response time T has elapsed, the vision system 14 acquisition and processing unit 17 generates a response signal 30 associated with the processing of the respective image 16.

Each response signal 30 comprises, if necessary, reject information 29 associated with the identifier 28.

The vision system 14 acquisition and processing unit 17 sends the response signals 30 to the electronic controller device 20.

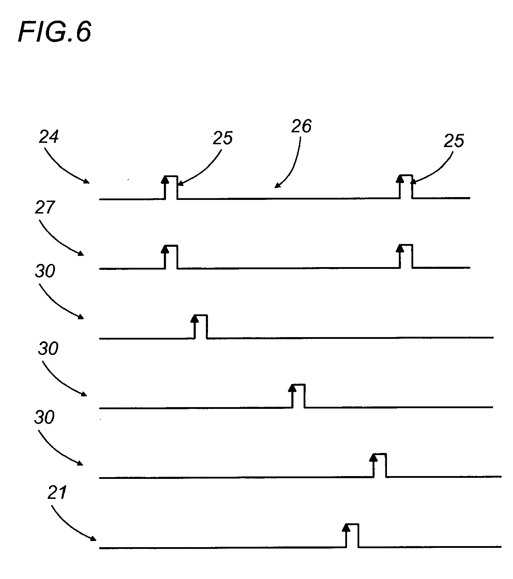

At this point, even if only one of the three response signals 30 comprises reject information 29 associated with the identifier 28, the electronic controller device 20 assigns the reject information 29 to the shift register 18 block 19 having the identifier 28, as shown in Figure 6.

Again in this embodiment, the shift register 18 shift command 21 may come before, after or simultaneously with each of the vision system 14 three response signals 30, since the reject information 29 present in the response signal 30 is not associated with a shift register 18 block 19 having a predetermined position 23, but is instead associated with the shift register 18 block 19 which has the identifier 28, whatever the position of the identifier in the shift register.

Advantageously, the generation of an identifier 28 and assignment of that identifier 28 to a shift register 18 block 19 allows the reject information 29 to be unambiguously associated with the block 19 having the identifier 28, leading to rejection of the defective individual article 2.

That also allows management of vision system 14 delays during machine 1 operation, separating the vision system 14 response time T from the shift register 18 shift command 21, provided that the vision system response time T is always within the same machine step 26, that is to say, between one trigger signal 27 and the next.

## Claims

1. A machine for making absorbent sanitary articles, such as nappies for babies or incontinence pads for adults, sanitary towels and the like, each article (2) comprising a plurality of components (4, 5) progressively positioned relative to each other and assembled along a production line (3) which comprises a plurality of operating stations (8, 9, 10) equipped with devices for cutting and/or sealing the materials, which the articles (2) are made up of, and a rejection station (13) downstream of the operating stations (8, 9, 10), the machine (1) comprising a programmable electronic controller device (20) comprising a shift register (18) and at least one optical inspection system (14) connected to the programmable electronic controller device (20); the programmable electronic controller device (20) generating a synchronism signal (24) having a train of consecutive pulses (25), two consecutive pulses (25) defining a machine step (26), a trigger signal (27) for activating the optical inspection system (14) in phase with the synchronism signal (24) and a shift command (21) of the shift register (18); the optical inspection system (14) receiving the trigger signal (27), capturing and processing at least one image (16) of the article (2) and, when a response time (T) has elapsed, generating a response signal (30), the machine being **characterised in that** in a machine step (26), the programmable electronic controller device (20) generates an identifier (28) which is uniquely associated with the article (2) inspected by the optical inspection system (14) and communicates to the optical inspection system (14) that identifier (28) with the trigger signal (27); the optical inspection system (14) receiving the identifier (28) and, when the response time (T) has elapsed, associating reject data (29) with the respective identifier (28) if the article (2) inspected is defective.

2. The machine according to claim 1, **characterised in that** the shift command (21) for the shift register (18) is independent of the response time (T) of the optical inspection system (14).

3. The machine according to claim 1 or 2, **characterised in that** the optical inspection system (4) comprises at least a video camera (15) for capturing at least one image of the article (2) and an image (16) acquisition and processing unit (17) connected to the video camera (15).

4. The machine according to any one of the claims 1 to 3, **characterised in that** the optical inspection system (14) is a vision system comprising at least one video camera (15).

5. The machine according to claim 3 or 4, **characterised in that** the image capturing video camera (15) is a linear video camera.

6. The machine according to any one of the claims 3 to 5, **characterised in that** the video camera (15) is activated a plurality of times in the same machine step (26).

7. The machine according to any one of the claims 3 to 5, **characterised in that** a plurality of video cameras (15) is activated in the same machine step (26).

8. A method of making absorbent sanitary articles, comprising a step of generating a machine (1) synchronism signal (24) by means of the programmable electronic controller device (20), the synchronism signal having a train of consecutive pulses (25) and two consecutive pulses (25) defining a machine step (26); a step of sending a trigger signal (27) to an optical inspection system (14) by means of the programmable electronic controller device (20), the optical inspection system (14) generating a response signal (30) when a response time (T) has elapsed; a step of applying a shift command (21) on a shift register (18) of the programmable electronic controller device (20), **characterised in that** it comprises a step of generating an identifier (28) for each machine step (26), the identifier (28) being uniquely associated with the article (2) inspected by the optical inspection system (14) and sending the identifier (28) with the trigger signal (27) to the optical inspection system (14) by means of the programmable electronic controller device (20); the optical inspection system (14) receiving the identifier (28) and, when the response time (T) has elapsed, associating reject data (29) with the respective identifier (28) if the article (2) inspected is defective.

9. The method according to claim 8, **characterised in that** it comprises the step of applying a shift command (21) on the shift register (18) of the programmable electronic controller device (20) independently of the response time (T) of the optical inspection system (14).

## Patentansprüche

1. Maschine zur Herstellung saugfähiger Hygieneartikel wie Windeln für Babys oder Inkontinenzeinlagen für Erwachsene, Hygienetücher und Dergleichen, wobei jeder Artikel (2) eine Vielzahl an Komponenten (4, 5) umfasst, die progressiv relativ zueinander positioniert und entlang einer Fertigungsanlage (3) zusammengefügt werden, die eine Vielzahl an Bearbeitungsstationen (8, 9, 10) umfasst, die mit Vorrichtungen zum Schneiden und/oder Siegeln der Materialien, aus denen die Artikel (2) bestehen, ausgestattet sind, und eine Aussonderungsstation (13), die nach den Bearbeitungsstationen (8, 9, 10) angeordnet ist, wobei die Maschine (1) ein programmierbares elektronisches Steuergerät (20) umfasst, umfassend ein Schieberegister (18) und mindestens ein optisches Inspektionssystem (14), verbunden mit dem programmierbaren elektronischen Steuergerät (20), wobei das programmierbare elektronische Steuergerät (20) ein Synchronlaufsignal (24) generiert, aufweisend eine Folge aufeinanderfolgender Impulsen (25), wobei zwei aufeinanderfolgende Impulse (25) einen Maschinenschritt (26) definieren, ein Auslösesignal (27) zur Aktivierung des optischen Inspektionssystems (14), das taktgleich mit dem Synchronlaufsignal (24) ist, und einen Umschaltbefehl (21) des Schieberegisters (18), wobei das optische Inspektionssystem (14) das Auslösesignal (27) empfängt und mindestens ein Bild (16) des Artikels (2) erfasst und verarbeitet und nach Ablauf einer Antwortzeit (T) ein Antwortsignal (30) generiert, wobei die Maschine **dadurch gekennzeichnet ist, dass** das programmierbare elektronische Steuergerät (20) in einem Maschinenschritt (26) einen Identifikator (28) generiert, der auf eindeutige Weise mit dem durch das optische Inspektionssystem (14) inspizierten Artikel (2) verknüpft ist und dem optischen Inspektionssystem (14) den Identifikator (28) mit dem Auslösesignal (27) übermittelt, wobei das optische Inspektionssystem (14) den Identifikator (28) empfängt und nach Ablauf der Antwortzeit (T) die Aussonderungsdaten (29) mit dem jeweiligen Identifikator (28) verknüpft, wenn der inspizierte Artikel (2) fehlerhaft ist.

2. Maschine nach Anspruch 1, **dadurch gekennzeichnet, dass** der Umschaltbefehl (21) für das Schieberegister (18) von der Antwortzeit (T) des optischen Inspektionssystems (14) unabhängig ist.

3. Maschine nach Anspruch 1 oder 2, **dadurch gekennzeichnet, dass** das optische Inspektionssystem (4) mindestens eine Videokamera (15) zum Erfassen von mindestens einem Bild des Artikels (2) und eine Einheit (17) zum Erfassen und Verarbeiten des Bilds (16) umfasst, die mit der Videokamera (15) verbunden ist.

4. Maschine nach einem der Ansprüche 1 bis 3, **dadurch gekennzeichnet, dass** das optische Inspektionssystem (14) ein Visionssystem ist, das mindestens eine Videokamera (15) umfasst.

5. Maschine nach Anspruch 3 oder 4, **dadurch gekennzeichnet, dass** die Bilderfassungsvideokamera (15) eine lineare Videokamera ist.

6. Maschine nach einem der Ansprüche 3 bis 5, **dadurch gekennzeichnet, dass** die Videokamera (15) viele Male im selben Maschinenschritt (26) aktiviert wird.

7. Maschine nach einem der Ansprüche 3 bis 5, **dadurch gekennzeichnet, dass** eine Vielzahl an Videokameras (15) im selben Maschinenschritt (26) aktiviert wird.

8. Verfahren zur Herstellung saugfähiger Hygieneartikel, umfassend einen Schritt zum Generieren eines Synchronlaufsignals (24) der Maschine (1) mittels des programmierbaren elektronischen Steuergeräts (20), wobei das Synchronlaufsignal eine Folge aufeinanderfolgender Impulse (25) aufweist und zwei aufeinanderfolgende Impulse (25) einen Maschinenschritt (26) definieren, einen Schritt zum Senden eines Auslösesignals (27) an ein optisches Inspektionssystem (14) mittels des programmierbaren elektronischen Steuergeräts (20), wobei das optische Inspektionssystem (14) ein Antwortsignal (30) generiert, wenn eine Antwortzeit (T) abgelaufen ist, einen Schritt zum Anwenden eines Umschaltbefehls (21) auf einem Schieberegister (18) des programmierbaren elektronischen Steuergeräts (20), **dadurch gekennzeichnet, dass** es einen Schritt zum Generieren eines Identifikators (28) für jeden Maschinenschritt (26) umfasst, wobei der Identifikator eindeutig mit dem vom Inspektionssystem (14) inspizierten Artikel (2) verknüpft ist, und zum Senden des Identifikators (28) mit dem Auslösesignal (27) an das optische Inspektionssystem (14) mittels des programmierbaren elektronischen Steuergeräts (20), wobei das optische Inspektionssystem (14) den Identifikator (28) empfängt und die Aussonderungsdaten (29) mit dem jeweiligen Identifikator (28) verknüpft, wenn der inspizierte Artikel (2) fehlerhaft ist, nachdem die Antwortzeit (T) abgelaufen ist.

9. Verfahren nach Anspruch 8, **dadurch gekennzeichnet, dass** es den Schritt zum Anwenden eines Umschaltbefehls (21) auf dem Schieberegister (18) des programmierbaren elektronischen Steuergeräts (20) umfasst, unabhängig von der Antwortzeit (T) des optischen Inspektionssystems (14).

## Revendications

1. Machine de fabrication d'articles sanitaires absorbants, tels que couches-culottes pour bébés ou couches pour adultes incontinents, serviettes hygiéniques ou analogues, chaque article (2) comprenant une pluralité de composants (4, 5) positionnés progressivement les uns par rapport aux autres et assemblés le long d'une ligne de production (3) qui comprend une pluralité de postes opérationnels (8, 9, 10) équipés de dispositifs destinés à couper et/ou à sceller les matériaux dont les articles (2) se composent, et un poste d'élimination (13) situé en aval des postes opérationnels (8, 9, 10), la machine (1) comprenant un dispositif de contrôle électronique programmable (20) comprenant un registre à décalage (18) et au moins un système d'inspection optique (14) relié au dispositif de contrôle électronique programmable (20) ; le dispositif de contrôle électronique programmable (20) générant un signal de synchronisation (24) ayant un train d'impulsions consécutives (25), deux impulsions consécutives (25) définissant un pas (26) de machine, un signal de déclenchement (27) destiné à activer le système d'inspection optique (14) en phase avec le signal de synchronisation (24) et un signal de changement (21) du registre à décalage (18) ; le système d'inspection optique (14) recevant le signal de déclenchement (27), capturant et traitant au moins une image (16) de l'article (2) et, lorsqu'un temps de réponse (T) s'est écoulé, générant un signal de réponse (30), la machine étant **caractérisée en ce que** dans un pas (26) de machine, le dispositif de contrôle électronique programmable (20) génère un identificateur (28) qui est uniquement associé à l'article (2) inspecté par le système d'inspection optique (14) et communique au système d'inspection optique (14) cet identificateur (28) et le signal de déclenchement (27) ; le système d'inspection optique (14) recevant l'identificateur (28) et, lorsque le temps de réponse (T) s'est écoulé, associant la donnée d'élimination (29) avec l'identificateur respectif (28) si l'article (2) inspecté est défectueux.

2. Machine selon la revendication 1, **caractérisée en ce que** le signal de changement (21) pour le registre à décalage (18) est indépendant du temps de réponse (T) du système d'inspection optique (14).

3. Machine selon les revendications 1 ou 2, **caractérisée en ce que** le système d'inspection optique (4) comprend au moins une caméra vidéo (15) pour capturer au moins une image de l'article (2) et une unité d'acquisition et de traitement (17) d'image (16) reliée à la caméra vidéo (15).

4. Machine selon l'une quelconque des revendications de 1 à 3, **caractérisée en ce que** le système d'inspection optique (14) est un système de vision comprenant au moins une caméra vidéo (15).

5. Machine selon les revendications 3 ou 4, **caractérisée en ce que** la caméra vidéo de capture d'image (15) est une caméra vidéo linéaire.

6. Machine selon l'une quelconque des revendications de 3 à 5, **caractérisée en ce que** la caméra vidéo (15) est activée plusieurs fois dans le même pas (26) de machine.

7. Machine selon l'une quelconque des revendications de 3 à 5, **caractérisée en ce qu'**une pluralité de caméras vidéo (15) est activée dans le même pas (26) de machine.

8. Procédé de fabrication d'articles sanitaires absorbants, comprenant une étape consistant à générer un signal de synchronisation (24) de la machine (1) au moyen d'un dispositif de contrôle électronique programmable (20), le signal de synchronisation ayant un train d'impulsions consécutives (25) et deux impulsions consécutives (25) définissant un pas (26) de machine ; une étape consistant à envoyer un signal de déclenchement (27) à un système d'inspection optique (14) au moyen du dispositif de contrôle électronique programmable (20), le système d'inspection optique (14) générant un signal de réponse (30) lorsqu'un temps de réponse (T) s'est écoulé ; une étape consistant à appliquer un signal de changement (21) sur un registre à décalage (18) du dispositif de contrôle électronique programmable (20), **caractérisé en ce qu'**il comprend une étape consistant à générer un identificateur (28) pour chaque pas (26) de machine, l'identificateur (28) étant uniquement associé à l'article (2) inspecté par le système d'inspection optique (14) et à envoyer l'identificateur (28) et le signal de déclenchement (27) au système d'inspection optique (14) au moyen du dispositif de contrôle électronique programmable (20) ; le système d'inspection optique (14) recevant l'identificateur (28) et, lorsque le temps de réponse (T) s'est écoulé, associant les données d'élimination (29) à l'identificateur correspondant (28) si l'article (2) inspecté est défectueux.

9. Procédé selon la revendication 8, **caractérisé en ce qu'**il comprend l'étape consistant à appliquer un signal de changement (21) sur le registre à décalage (18) du dispositif de contrôle électronique programmable (20) indépendamment du temps de réponse (T) du système d'inspection optique (14).
